(19) Europäisches Patentamt / European Patent Office / Office européen des brevets

(11) **EP 2 459 531 B1**

(12) **EUROPEAN PATENT SPECIFICATION**

(45) Date of publication and mention of the grant of the patent: **11.09.2019 Bulletin 2019/37**

(21) Application number: **10742739.5**

(22) Date of filing: **30.07.2010**

(51) Int Cl.: ***C07D 207/16*** $^{(2006.01)}$ ***A61K 31/4015*** $^{(2006.01)}$ ***A61K 9/16*** $^{(2006.01)}$

(86) International application number: **PCT/EP2010/004689**

(87) International publication number: **WO 2011/012322 (03.02.2011 Gazette 2011/05)**

(54) **GRANULATE COMPRISING VILDAGLIPTIN AND PROCESS FOR ITS PREPARATION**

GRANULAT ENTHALTEND VILDAGLIPTIN UND VERFAHREN ZU DESSEN HERSTELLUNG

GRANULÉS COMPRENANT DE LA VILDAGLIPTINE ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **31.07.2009 SI 200900216**
**16.10.2009 SI 200900295**

(43) Date of publication of application: **06.06.2012 Bulletin 2012/23**

(73) Proprietor: **KRKA, D.D., Novo Mesto**
**8501 Novo mesto (SI)**

(72) Inventors:
- **ZUPET, Rok**
  **1000 Ljubljana (SI)**
- **SMODIS, Janez**
  **8323 Ursna sela (SI)**
- **STROPNIK, Tadej**
  **8321 Brusnice (SI)**
- **ANDERLUH, Marko**
  **1000 Ljubljana (SI)**
- **VRBINC, Miha**
  **8000 Novo Mesto (SI)**
- **VRECER, Franc**
  **8351 Straza (SI)**
- **PECAVAR, Anica**
  **8000 Novo Mesto (SI)**
- **KUKEC, Simon**
  **28050 Madrid (ES)**

(74) Representative: **Hoefer & Partner Patentanwälte mbB**
**Pilgersheimer Straße 20**
**81543 München (DE)**

(56) References cited:

| | |
|---|---|
| **WO-A1-00/34241** | **WO-A1-2004/092127** |
| **WO-A2-2006/135693** | **WO-A2-2007/019255** |
| **WO-A2-2007/041053** | **WO-A2-2007/078726** |
| **WO-A2-2009/121945** | **US-A1- 2008 167 479** |

- **VILLHAUER E B ET AL: "1-ÄÄ(3-HYDROXY-1-ADAMANTYL)AMINOÜAC ETYLÜ- 2-CYANO-(S)-PYRROLIDINE: A POTENT, SELECTIVE, AND ORALLY BIOAVAILABLE DIPEPTIDYL PEPTIDASE IV INHIBITOR WITH ANTIHYPERGLYCEMIC PROPERTIES", JOURNAL OF MEDICINAL CHEMISTRY, AMERICAN CHEMICAL SOCIETY, WASHINGTON, US, vol. 46, no. 13, 1 January 2003 (2003-01-01), pages 2774-2789, XP001165747, ISSN: 0022-2623, DOI: DOI:10.1021/JM030091L**
- **Mieczyslaw Makosza and Michal Fedorynski: "Phase Transfer Catalysis" In: "Encyclopedia of Catalysis", 15 July 2002 (2002-07-15), John Wiley & Sons, Inc., XP002616580, pages 1-59, pages 9 and 10, "Alkylation of N-Anions"**
- **SALVATORE R N ET AL: "Synthesis of secondary amines", TETRAHEDRON, ELSEVIER SCIENCE PUBLISHERS, AMSTERDAM, NL, vol. 57, no. 37, 10 September 2001 (2001-09-10), pages 7785-7811, XP004304423, ISSN: 0040-4020, DOI: DOI:10.1016/S0040-4020(01)00722-0**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **Misc.: "Handbook of Pharmaceutical Granulation Technology" In: "Handbook of Pharmaceutical Granulation Technology", 2 January 2005 (2005-01-02), XP055435065, pages 1-624,**

Remarks:

The file contains technical information submitted after the application was filed and not included in this specification

## Description

### FIELD OF THE INVENTION

The present invention relates to the use of vildagliptin or its pharmaceutically acceptable salts for the preparation of solid oral dosage forms.

### BACKGROUND OF THE INVENTION

Vildagliptin is an active pharmaceutical substance with an empirical formula of $C_{17}H_{25}N_3O_2$ and a molecular weight of 303.40 g/mol. Vildagliptin is the international commonly accepted name for (2"S)-1-[[(3-hydroxytricyclo[3.3.1.1' ]dec-1-yl)amino]acetyl]-2- pyrrolidine carbonitrile and has the structure of formula **(I)**.

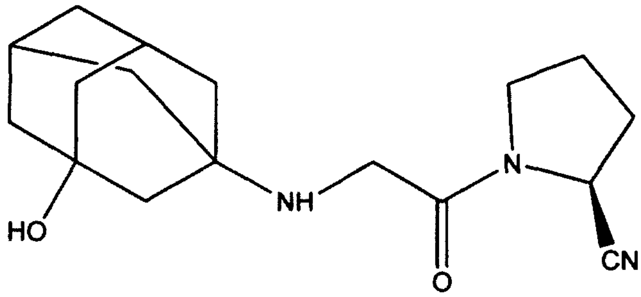

**(I)**

Vildagliptin is a dipeptidyl peptidase IV (DPP-IV) inhibitor and is disclosed in EP 1137635. The '635 patent discloses a synthesis of vildagliptin using the synthetic process represented in Scheme 1.

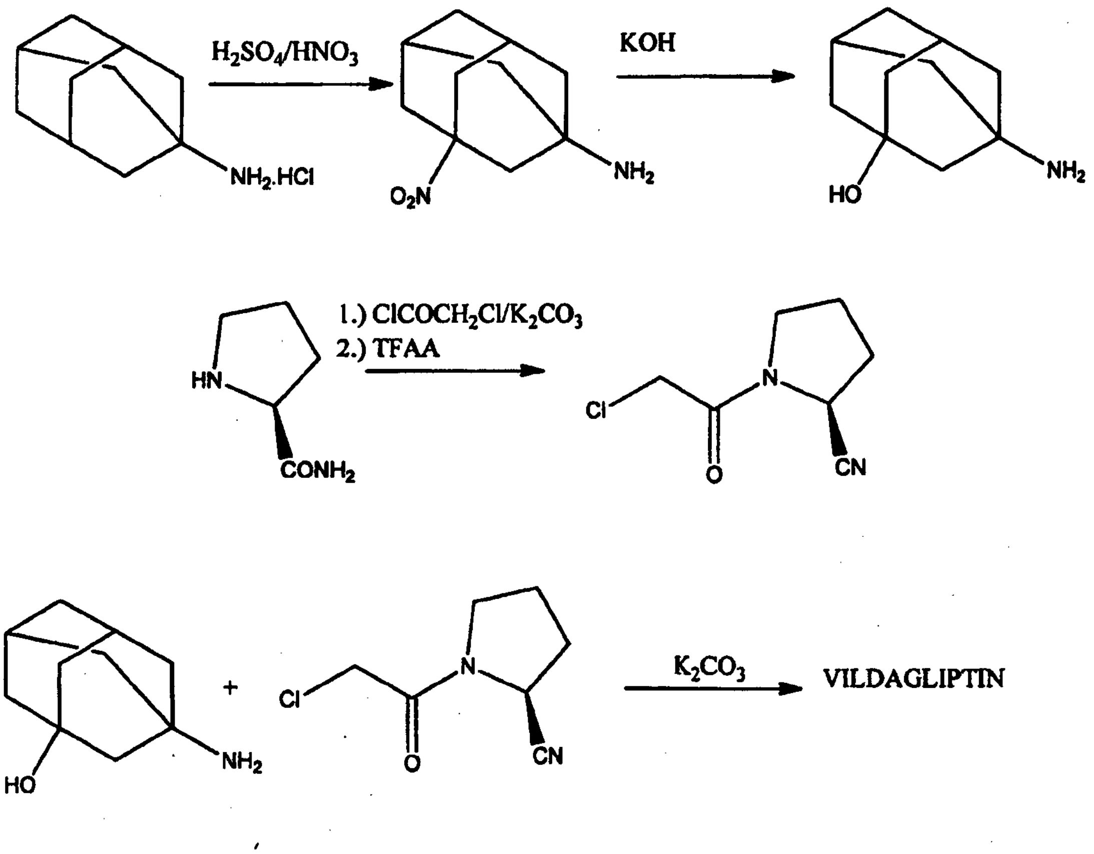


Scheme 1

Vildagliptin can exist as the (2S) and (2R) enantiomeric form. The stereoisomer with the desired biological activity is the (2S) enantiomer. Accordingly, it is desirable to synthesize (2S)- vildagliptin with high stereochemical purity. A process that yields vildagliptin with a high enantiomeric purity is disclosed in International Patent Publication WO 2004/092127. This reference discloses compositions containing from 95% to 99.99% of (2S)-vildagliptin.

WO 2007/078726 A2 discloses pharmaceutical compositions comprising fixed-dose combinations of a dipeptidyl peptidase-4 inhibitor and metformin, methods of preparing such pharmaceutical compositions, and methods of treating Type 2 diabetes with such pharmaceutical compositions.

WO 2009/121945 A2 (International publication date: 08.10.2009) relates to pharmaceutical compositions comprising fixed dose combinations of a DPP-4 inhibitor drug and a partner drug, processes for the preparation thereof, and their use to treat certain diseases.

WO 2007/019255 A2 relates to novel salt forms of vildagliptin (LAF237), i.e. salt forms of (S)-1-[(3-hydroxy-1-adamantyl)amino]-acetyl-2-cyano-pyrrolidine.

WO 2007/041053 A2 relates to a formulation comprising a dipeptidyl-peptidase IV (DPP-IV) inhibitor preferably vildagliptin and metformin, to tablets comprising such formulations and to processes for the preparation thereof.

WO 00/34241 A1 describes several compounds inhibiting DPP-IV (dipeptidyl-peptidase-IV) activity. These compounds are therefore indicated in WO 00/34241 A1 for use as pharmaceuticals in inhibiting DPP-IV and in the treatment of conditions mediated by DPP-IV, such as non-insulin-dependent diabetes mellitus, arthritis, obesity, osteoporosis and further conditions of impaired glucose tolerance.

CN 101234105 (A) relates to medical composition containing metformin and Vildagliptin and a preparation method thereof.

CN 101181264 (A) describes pharmaceutical compositions taking metformin hydrochloride and vildagliptin as active component.

However, there remains a need for a process for preparing vildagliptin with high chemical and enantiomeric purity.

Described is a process, wherein vildagliptin is prepared in a quick and reliable way, with high yield as well as chemical and enantiomerical purity.

## SUMMARY OF THE INVENTION

Described is a method of making vildagliptin including the step of reacting a 2-acetyl substituted (S)-1-(acetylpyrrolidin-2-carbonitrile of formula (II)

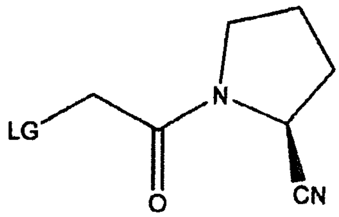

(II),

preferably (S)-1-(2-chloroacetyl)pyrrolidin-2-carbonitrile and 3-amino-1-adamantole of formula (III) in the presence of a phase transfer catalyst. The reaction mixture may be comprised by a first and a second phase, wherein the first phase designates the liquid phase and the second phase preferably designates the solid phase, optionally of more different compounds, un-dissolved in the first phase solvent.

Described is a method of making vildagliptin including the step of reacting a 2-acetyl substituted (S)-1-(acetylpyrrolidin-2-carbonitrile of formula (II)

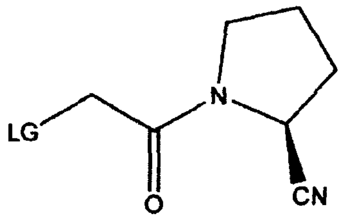

(II),

preferably (S)-1-(2-chloroacetyl)pyrrolidin-2-carbonitrile, and 3-amino-1-adamantole (III) in the presence of a base and a phase transfer catalyst in a reaction system having a first and a second phase, wherein the first phase includes a first solvent that is chosen from the group of nitriles, such as acetonitrile, ketones, such as acetone, 2-butanone, 2- or 3-pentanone; ethers, e.g. a $C_2$-$C_6$ cycloalkyl ether, preferably tetrahydrofuran; or a $C_3$ to $C_6$ ester, such as ethyl acetate, isopropyl acetate, isobutyl acetate, and mixtures of them,

and the second phase includes the solid interface of an inorganic base, insoluble in the first solvent, for example KOH, NaOH, LiOH, $K_2CO_3$, $Na_2CO_3$, $Cs_2CO_3$ etc, especially $K_2CO_3$.

Described is a method of making vildagliptin including the step of reacting a 2-acetyl substituted (S)-1-(acetylpyrrolidin-2-carbonitrile of formula (II)

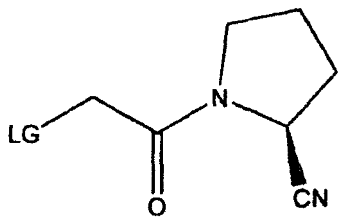

(II),

preferably (S)-1-(2-chloroacetyl)pyrrolidin-2-carbonitrile, and 3-amino-1-adamantole (III) in the presence of benzyl triethyl ammonium chloride in a reaction system having a first and a second phase, wherein the first phase includes a first solvent that is chosen from the group of nitriles, such as acetonitrile, ketones, such as acetone, 2-butanone, 2- or 3-pentanone; ethers, e.g. a $C_2$-$C_6$ cycloalkyl ether, preferably tetrahydrofuran; or a $C_3$ to $C_6$ ester, such as ethyl acetate, isopropyl acetate, isobutyl acetate, and mixtures of them,
and the second phase includes the solid interface of an inorganic base, insoluble in the first solvent, for example KOH, NaOH, LiOH, $K_2CO_3$, $Na_2CO_3$, $Cs_2CO_3$ etc, especially $K_2CO_3$.

## DETAILED DESCRIPTION OF THE INVENTION

Described is a method of making vildagliptin including the step of reacting a 2-acetyl substituted (S)-1-(acetylpyrrolidin-2-carbonitrile of formula (II)

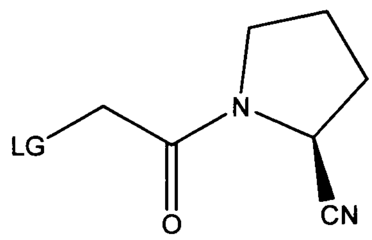

(II),

preferably (S)-1-(2-chloroacetyl)pyrrolidin-2-carbonitrile and 3-amino-1-adamantole of formula (III) in the presence of a phase transfer catalyst. The reaction mixture may be comprised by a first and a second phase, wherein the first phase designates the liquid phase and the second phase preferably designates the solid phase, optionally of two or more different compounds, un-dissolved in the first phase solvent. Optionally a base is present.

In one of the embodiments, the first and second phases include first and second solvents, respectively, which are substantially immiscible in each other so that, when combined in a reaction vessel, a two-phase system is formed. Solvents are substantially immiscible in each other when equal volumes of them are mixed together, a two-phase system is formed in which the volume of the two phases is essentially equal. Preferably, substantially immiscible solvents are soluble in each other to the extent of about 1% (weight basis) or less.

In another embodiment of the present invention, the first phase designates the liquid phase and the second phase designates the solid phase, optionally composed of two or more different compounds, un-dissolved in the first phase solvent. Preferably, the second phase includes the solid interface of an inorganic base, insoluble in the first solvent, for example KOH, NaOH, LiOH, $K_2CO_3$, $Na_2CO_3$, $Cs_2CO_3$ etc, especially $K_2CO_3$.

LG in the compound of formula (II) represents a leaving group for nucleophilic reactions, such as Cl, Br, I, p-Me-$C_6H_4$-$SO_2O$-, p-$NO_2$-$C_6H_4$-$SO_2O$-, $CH_3SO_2O$-, or $CF_3SO_2O$-, preferably Cl.

When compounds of formula (II) and (III) are to be reacted in the presence of an inorganic base, one or more of the reactants are dissolved in a first solvent and one or more reactants are dissolved in a second solvent, or remain un-dissolved in the first solvent without the presence of a second solvent. Because at least one of the reactants is essentially insoluble in the first solvent, a two-phase system is formed and reaction occurs at the interface between the two phases.

The rate of such an interfacial reaction can be greatly increased by use of a phase transfer catalyst (PTC).

Several classes of compounds are known to be capable of acting as phase transfer catalysts, for example quaternary ammonium compounds, such as tetrabutylammonium hydrogen sulfate, or benzyl triethyl ammonium chloride or bromide; phosphonium compounds, crown ethers etc. Benzyl triethyl ammonium chloride is a preferred PTC for use in the practice of the present invention.

In one embodiment of the synthetic method described
herein, a 2-acetyl substituted (S)-1-(acetylpyrrolidin-2-carbonitrile of formula (II)

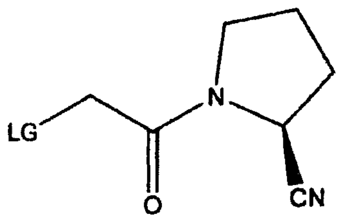

(II),

preferably (S)-1-(2-chloroacetyl)pyrrolidin-2-carbonitrile, and 3-amino-1-adamantole (III), together with a inorganic base, are reacted in the presence of a phase transfer catalyst in a reaction system having a first and a second phase, wherein the first phase includes a first solvent that is chosen from the group of nitriles, such as acetonitrile, ketones, such as acetone, 2-butanone, 2- or 3-pentanone; ethers, e.g. a $C_2$-$C_6$ cycloalkyl ether, preferably tetrahydrofuran; or a $C_3$ to $C_6$ ester, such as ethyl acetate, isopropyl acetate, isobutyl acetate, and mixtures of them,

and the second phase includes the solid interface of an inorganic base, insoluble in the first solvent, for example KOH, NaOH, LiOH, $K_2CO_3$, $Na_2CO_3$, $Cs_2CO_3$ etc, especially $K_2CO_3$.

Also to be provided is a process, wherein (S)-1-(2-chloroacetyl)pyrrolidin-2-carbonitrile, and 3-amino-1-adamantole (III), together with $K_2CO_3$, are reacted in the presence of benzyl triethyl ammonium chloride in a reaction system having a first and a second phase, wherein the first phase includes a first solvent that is acetonitrile and the second phase includes the solid interface of $K_2CO_3$.

The base is present in an amount between about 1 and about 10 molar equivalents, preferably in an amount between 3 and 5 equivalents, relative to the number of moles of compound of formula (II).

The compound of formula (II) may be prepared as e.g. disclosed in EP 1137635 or EP1620396. It may also be prepared in the form of an acid addition salt, e.g. such as the 4-acetamidobenzoate, acetate, adipate, alginate, 4-aminosalicylate, ascorbate, aspartate, benzenesulfonate, benzoate, butyrate, camphorate, camphorsulfonate, carbonate, cinnamate, citrate, cyclamate, cyclopentanepropionate, decanoate, 2,2- dichloroacetate, digluconate, dodecylsulfate, ethane-1 ,2-disulfonate, ethanesulfonate, formate, fumarate, galactarate, gentisate, glucoheptanoate, gluconate, glucuronate, glutamate, glycerophosphate, glycolate, hemisulfate, heptanoate, hexanoate, hippurate, hydrochloride, hydrobromide, hydroiodide, 2-hydroxyethanesulfonate, isobutyrate, lactate, lactobionate, laurate, malate, maleate, malonate, mandelate, methanesulfonate, naphthalene-1 ,5-disulfonate, 2-naphthalenesulfonate, nicotinate, nitrate, octanoate, oleate, orotate, oxalate, 2-oxoglutarate, palmitate, pamoate, pectinate, persulfate, 3- phenylpropionate, phosphate, picrate, pidolate (L-pyroglutamate), pivalate, propionate, salicylate, sebacate, hydrogen sebacate, stearate, succinate, sulfate, tannate, tartrate, hydrogen tartrate, thiocyanate, tosylate, and undecanoate. In the case of polybasic acids, there are included acids in which all the acidic protons are removed as well as those in which one or, for example in the case of citrate, two protons are removed, as for example in the case of hydrogensulfate, hydrogenmalonate, hydrogenfumarate, hydrogenmalate, hydrogenmaleate, hydrogentartrate and hydrogengalactarate salt of compound (II). The compounds of formula (II), in particular (S)-1-(2-chloroacetyl)pyrrolidin-2-carbonitrile are preferably isolated with an optical purity of between 95.00 % (w/w) to 99.95 % (w/w), and a purity between 98.00 % (w/w) to 99.95 % (w/w). The water content is preferably less than 0.5 % (w/w), more preferably less than 0.1 % (w/w).

The purity and enantiomeric purity of (S)-1-(2-chloroacetyl)pyrrolidin-2-carbonitrile and compounds (II) in general may be determined with the following HPLC method:

<u>Method for chromatographic purity</u>

| | |
|---|---|
| **Column:** | Ascentis Express C8, 2.7 μm particles, 100 x 4.6 mm i.d., |
| **Eluent A:** | 0.02M $K_2HPO_4$, pH adjusted to 8.0 with phosphoric acid |
| **Eluent B:** | acetonitrile |

**Gradient:**

| Time (min) | % v/v A | % v/v B |
|---|---|---|
| 0 | 95 | 5 |
| 6.5 | 95 | 5 |
| 17 | 35 | 65 |
| 19 | 35 | 65 |
| 20 | 95 | 5 |

| | |
|---|---|
| **Post time:** | 4 min |
| **Flow-rate:** | 1.2 ml/min |
| **Detection:** | UV, 210 nm |
| **Injection volume:** | 5 µl |
| **Column temperature:** | 30°C |
| **Diluent:** | 50% methanol |

**Sample preparation:**

Accurately weigh about 20 mg of sample into 20 ml volumetric flask, dissolve and dilute to volume with diluent.

**Calculation:**

Use area per cent method. Do not integrate solvent peaks.

Method for enantiomeric purity

| | |
|---|---|
| **Column:** | Chiralpak IA, 5 µm particles, 250 x 4.6 mm i.d., |
| **Mobile phase:** | methanol : ethanol in the ratio 50 : 50 |
| **Flow-rate:** | 0.7 ml/min |
| **Detection:** | UV, 215 nm |
| **Injection volume:** | 20 µl |
| **Column temperature:** | 25°C |
| **Diluent:** | ethanol |

**Sample preparation:**

Accurately weigh about 20 mg of sample into 20 ml volumetric flask, dissolve and dilute to volume with diluent.

**Calculation:**

$$\%\ \text{R-enantiomer} = \frac{\text{area}_{\text{S-enantiomer}}}{\sum(\text{area}_{\text{S-enantiomer}} + \text{area}_{\text{R-enantiomer}})} \times 100$$

In another embodiment of the present invention, compound (II) may be prepared by reacting a compound of formula (IV)

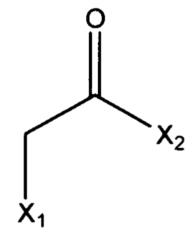

(IV),

wherein independently of each other,

$X_1$ is halogen, such as Cl, Br, I, preferably Cl; p-Me-$C_6H_4$-$SO_2O$-, p-$NO_2$-$C_6H_4$-$SO_2O$-, $CH_3SO_2O$-, or $CF_3SO_2O$-; $X_2$ is halogen, OH, O-C(=O)-$CH_2X_3$, -$OSO_2$-($C_{1-8}$) alkyl or -O-$SO_2$-(aryl), and $X_3$ is halogen,

with L-prolinamide to form a compound of formula (V)

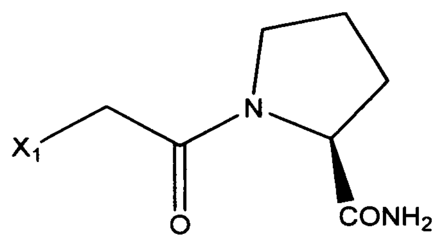

(V)

in the presence of dimethylacetamide (DMA), followed by reacting the resultant compound (V) with or without isolation of compound (V), with a dehydration agent, such as trifluoroacetic anhydride, cyanuric acid halide, preferably cyanuric acid chloride, Vilsmeier reagent (chloroiminium ion) or Gold reagent ($Me_2NCH=NCH=NMe_2^+Cl^-$, prepared from cyanuric chloride and dimethylformamide) to form the compound of formula (II). Optionally, a base (organic or inorganic) is present, for example KOH, NaOH, LiOH, $K_2CO_3$, $Na_2CO_3$, $Cs_2CO_3$ etc, especially $K_2CO_3$.

The compound of formula (V) can, if isolated, be isolated in the form of an acid addition salt, such as in the form of 4-acetamidobenzoate, acetate, adipate, alginate, 4-aminosalicylate, ascorbate, aspartate, benzenesulfonate, benzoate, butyrate, camphorate, camphorsulfonate, carbonate, cinnamate, citrate, cyclamate, cyclopentanepropionate, decanoate, 2,2- dichloroacetate, digluconate, dodecylsulfate, ethane-1 ,2-disulfonate, ethanesulfonate, formate, fumarate, galactarate, gentisate, glucoheptanoate, gluconate, glucuronate, glutamate, glycerophosphate, glycolate, hemisulfate, heptanoate, hexanoate, hippurate, hydrochloride, hydrobromide, hydroiodide, 2-hydroxyethanesulfonate, isobutyrate, lactate, lactobionate, laurate, malate, maleate, malonate, mandelate, methanesulfonate, naphthalene-1 ,5-disulfonate, 2-naphthalenesulfonate, nicotinate, nitrate, octanoate, oleate, orotate, oxalate, 2-oxoglutarate, palmitate, pamoate, pectinate, persulfate, 3- phenylpropionate, phosphate, picrate, pidolate (L-pyroglutamate), pivalate, propionate, salicylate, sebacate, hydrogen sebacate, stearate, succinate, sulfate, tannate, tartrate, hydrogen tartrate, thiocyanate, tosylate, and undecanoate. In the case of polybasic acids, there are included acids in which all the acidic protons are removed as well as those in which one or, for example in the case of citrate, two protons are removed, as for example in the case of hydrogensulfate, hydrogenmalonate, hydrogenfumarate, hydrogenmalate, hydrogenmaleate, hydrogentartrate and hydrogengalactarate salt, or in the form of a base as depicted in formula (V). The compounds of formula (V), in particular (S)-1-(2-chloroacetyl)pyrrolidine-2-carboxamide are preferably isolated with an optical purity of between 95.00 % (w/w) to 99.95 % (w/w), and a purity between 98.00 % (w/w) to 99.95 % (w/w). The water content is preferably less than 0.5 % (w/w), more preferably less than 0.1 % (w/w), with the total content of amino acids less than 0.3 % (w/w).

L-prolinamide is commercially available with chemical and enantiomerical purities above 95 % (w/w), preferably above 99 % (w/w), more preferably above 99.9 % (w/w). The water content of L-prolinamide is preferably less than 0.5 % (w/w), more preferably less than 0.1 % (w/w), the total content of amino acids less than 0.3 % (w/w) and the total contents of L-glutamate, proline and (S)-1-pyrroline-5-carboxylate less than 0.1 % (w/w) each.

3-amino-1-adamantole of formula (III) is commercially available with chemical and enantiomerical purities above

95 % (w/w), preferably above 99 % (w/w), more preferably above 99.9 % (w/w). The water content of 3-amino-1-adamantole is preferably less than 0.5 % (w/w), more preferably less than 0.1 % (w/w), most preferably less than 0.05 % (w/w).

The compounds of formula (II) and (III), or their salts, in solid form or their solutions, and the inorganic base, are combined in any order to form a two-phase reaction system that has a first and a second phase. The combining can be in any suitable vessel that is equipped with means for vigorous agitation of the reaction system to maximize the interfacial area between the two phases. The combining can be at any temperature from about 20 °C to about 95 °C, preferably at about 50 °C. The reaction is allowed to proceed in the two phase system for a time that the skilled artisan will know to adjust according to the reaction temperature.

After the reaction time and to facilitate phase separation, the reaction system is allowed to cool, preferably to a temperature of about 5 °C to about 30 °C and the first and second phases are separated. If the second phase is solid, the solution of the first solvent is filtered to separate it from the second phase. If desired, the liquid phase(s) can be extracted one or more times and the combined with the first phase. The solvent is removed from the separated first phase, preferably by evaporation, especially at reduced pressure, to afford a crude residue.

The crude residue may be dissolved or dispersed in a suitable solvent and re-crystallized or macerated to the purity desired. Suitable solvents are e.g. acetone, isopropyl alcohol, isobutyl alcohol, 2-butanone, ethyl acetate or mixtures thereof, preferably a mixture comprising ethyl acetate and isopropyl alcohol. Vildagliptin (I) may also be transformed into one of its acid addition salts, as e.g. disclosed in EP1912938, either after crystallization of the crude product or directly from the reaction mixture, optionally prior to the evaporation of the volatile components, and optionally transformed back to its base form as depicted in formula (I), in order to achieve further purification.

Vildagliptin may, by adjustment of crystallization parameters, be prepared in any desired average particle size, from 3 to 600 micrometers, preferably from 20 to 150 micrometers or from 250 to 350 micrometers, depending on the technology applied for the preparation of the final solid oral dosage form comprising vildagliptin (I).

In another embodiment, the present description provides fine particle size or "micronized" vildagliptin including a plurality of vildagliptin particles wherein the average particle size is about 3 μm to about 25 μm and/or 10 volume percent or less of the plurality of particles have a particle diameter equal to or greater than about 50 micrometers.

Micronized vildagliptin including a plurality of vildagliptin particles can be obtained by comminution using a fluid energy mill, wherein the average particle size produced is about 3 μm to about 25 μm and/or 10 volume percent or less of the plurality of particles have a particle diameter equal to or greater than about 30 μm.

A fluid energy mill, or "micronizer", is an especially preferred type of mill for its ability to produce particles of small size in a narrow size distribution, i. e., micronized material. As those skilled in the art are aware, micronizers use the kinetic energy of collision between particles suspended in a rapidly moving fluid (typically air) stream to cleave the particles. An air jet mill is a preferred fluid energy mill. The suspended particles are injected under pressure into a recirculating particle stream. Smaller particles are carried aloft inside the mill and swept into a vent connected to a particle size classifier such as a cyclone. The feedstock should first be milled to about 30 μm to 100 μm which may be done using a conventional ball, roller, or hammer mill.

The starting material, in particular the material comprising or consisting of vildagliptine and to be adjusted in size, for example by milling, may have an average particle size of about 200 μm to 600 μm.

The material is fed into the micronization system in a controlled feed rate by means of a screw feeder or a vibratory feeder. The air jet mill is operated with controlled air pressures. For the Microgrinding MC-500 KX, the feed rate is 40-80 kg/hr, the Feed air pressure is 6-8.5 bar and the grinding air is 3-6 bar.

Micronization can also be accomplished with a pin mill. The starting material may have an average particle size of about 20-100 microns. The material is fed into the mill system in a controlled feed rate by means of a screw feeder or a vibratory feeder. The mill is operated with controlled speed. For the Alpine UPZ 160, the feed rate is 60-75 kg/hr, the mill speed is 7,000-15,000 rpm.

Micronized vildagliptin can be used to make pharmaceutical compositions that can be in the form of solid oral dosage forms, for example compressed tablets. Compressed tablets can be made by dry, melt or wet granulation methods as is known in the art, or by direct compression. In addition to the pharmaceutically active agent or drug, compressed tablets contain a number of pharmacologically inert ingredients, referred to as excipients. Some excipients allow or facilitate the processing of the drug into tablet dosage forms. Other excipients contribute to proper delivery of the drug by, for example, facilitating disintegration.

In particular, described is a process of making vildagliptin or an acid addition salt thereof, which process further comprises:

i) isolating vildagliptin after having reacted a 2-acetyl substituted (S)-1-(acetylpyrrolidin-2-carbonitrile of formula (II) and 3-amino-1-adamantole of formula (III) in the presence of a phase transfer catalyst, ii) optionally purifying the isolated vildagliptin, preferably subjecting the isolated vildagliptin to a re-crystallization and/or to a maceration, iii) optionally transforming the isolated vildagliptin or the purified vildagliptin in an acid addition salt of vildagliptin, iv)

mixing the isolated vildagliptin or the purified vildagliptin or the acid addition salt of vildagliptin with one or more additional ingredients to produce a mixture comprising vildagliptin or an acid addition salt thereof, v) subjecting the mixture comprising vildagliptin or an acid addition salt thereof to granulation, and vi) optionally compressing the granulated vildagliptin to a comprimate comprising vildagliptin or an acid addition salt thereof.

In the context of the present invention, the term "acid addition salt of vildagliptin" may have in particular the meaning of a pharmaceutically acceptable acid addition salt of vildagliptin and the term "salt of vildagliptin" may have in particular the meaning of a pharmaceutically acceptable salt of vildagliptin. The term "pharmaceutically acceptable (acid addition) salt" refers in the context of the present application in particular to those salts which are, within the scope of sound medical judgment, suitable for use in contact with the tissues of human beings or animals without excessive toxicity, irritation, allergic response, or other problem or complication, commensurate with a reasonable benefit/risk ratio. A pharmaceutically acceptable salt of vildagliptin can be in particular a hydrochloride, hydrobromide, hydroiodide, hemisulfate, sulfate, lactate, carbonate, 4-acetamidobenzoate, acetate, adipate, alginate, ascorbate, aspartate, benzenesulfonate, benzoate, butyrate, camphorate, camphorsulfonate, cinnamate, citrate, cyclamate, cyclopentanepropionate, decanoate, 2,2-dichloroacetate, digluconate, dodecylsulfate, ethane-1 ,2-disulfonate, ethane- sulfonate, formate, fumarate, galactarate, gentisate, glucoheptanoate, gluconate, glucuronate, glutamate, glycerophosphate, glycolate, heptanoate, hexanoate, hippurate, 2-hydroxyethanesulfonate, isobutyrate, laurate, malate, maleate, malonate, mandelate, methanesulfonate, nicotinate, nitrate, octanoate, oleate, orotate, oxalate, 2-oxoglutarate, palmitate, pamoate, pectinate, 3-phenylpropionate, phosphate, L-pyroglutamate, pivalate, propionate, salicylate, sebacate, hydrogen sebacate, stearate, succinate, tannate, tartrate, hydrogen tartrate, tosylate, or undecanoate salt.

Additional ingredient(s) for mixing with vildagliptin or an acid addition salt thereof for producing a mixture comprising vildagliptin or an acid addition salt thereof can be for example further active ingredient(s) and/or excipient(s), for example at least one of diluent(s), binder(s), disintegrant(s), antioxidant(s), pH modifier(s), glidant(s), adsorbent(s), etc..

In particular, the granulation can be or can comprise a granulation procedure selected from the group consisting of melt granulation, wet granulation, in particular moisture activated granulation, preferably non-aqueous moisture activated granulation, dry granulation and combinations of these granulation procedures.

The present invention also provides a process of making a granulate comprising
vildagliptin or an acid addition salt thereof, which process comprises: a) providing a material comprising or consisting of vildagliptin or an acid addition salt thereof, b) mixing the material comprising or consisting of vildagliptin or an acid addition salt thereof with one or more additional ingredients to produce a mixture comprising vildagliptin or an acid addition salt thereof, c) subjecting the mixture comprising vildagliptin or an acid addition salt thereof to granulation, which granulation is wet granulation, in particular moisture activated granulation, preferably non-aqueous moisture activated granulation, said wet granulation being wet granulation with liquid(s) free of gelatine, and said wet granulation using solvent selected from alcohols with 1 to 4 carbon atoms, and mixtures thereof, and the binder for use in wet granulation being selected from hydroxymethylcellulose, hydroxyethylcellulose, hydroxypropylcellulose, and mixtures thereof.

Preferably, the material comprising or consisting of vildagliptin or an acid addition salt thereof is obtainable according to a process comprising reacting a 2-acetyl substituted (S)-1-(acetylpyrrolidin-2-carbonitrile of formula (II) and 3-amino-1-adamantole of formula (III) in the presence of a phase transfer catalyst. The wet granulation, in particular a moisture activated granulation, preferably a non-aqueous moisture activated granulation is carried out with granulation liquid(s) free of gelatine. During a wet granulation with liquid free of gelatine, the mixture comprising vildagliptin or an acid addition salt thereof is contacted with granulation liquid(s) which are free of gelatine. Surprisingly, wet granulations with liquid(s) free of
gelatine are advantageous when producing pharmaceutical compositions on industrial scale.

The process of making vildagliptin or an acid addition salt thereof may further comprise: adjusting the average particle size, preferably reducing the average particle size, of the isolated vildagliptin or the purified vildagliptin or of the acid addition salt of vildagliptin or of the mixture comprising vildagliptin or an acid addition salt thereof, in particular before carrying out the granulation procedure.

The process of making a granulate comprising or consisting of vildagliptin or an acid addition salt thereof may further comprise: adjusting the average particle size, preferably reducing the average particle size, of the material comprising or consisting of vildagliptin or an acid addition salt thereof or of the mixture comprising vildagliptin or an acid addition salt thereof, in particular before carrying out granulation.

The average particle size can be adjusted such, preferably reduced such that the resulting particles with adjusted, preferably reduced, average particle size have an average particle size in the range of about 3 $\mu$m to about 25 $\mu$m and/or that 10 volume percent or less of the resulting plurality of particles with adjusted, preferably reduced, average particle size have a particle diameter equal to or greater than about 50 micrometers.

A step of adjusting, preferably reducing, the average particle size may comprise one or more milling steps. Preferably the one or more milling steps being carried out using one or more mills selected from the group consisting

of pin mill, fluid energy mill, ball mill, roller mill or hammer mill or combinations of these mills.

A preferred wet granulation process is or comprises moisture activated granulation, in particular non-aqueous moisture activated granulation. In yet another embodiment of the present invention, wet granulation process comprises moisture activated granulation.

It is preferred to add during granulation, in particular during wet granulation, in particular during moisture activated granulation, especially during non-aqueous moisture activated granulation less than 10 wt %, preferably between 0.1 and 8.5 wt %, preferably between 0.5 and 8.5 wt %, in particular between 3.5 than 8,5 wt % of non-aqueous liquid, more preferably between 4 and 8 wt % based on the total weight of vildagliptin subjected to granulation or based on the total weight of acid addition salt of vildagliptin subjected to granulation.

Furthermore, it is preferred to add during non-aqueous moisture activated granulation for example less than 0.5 wt %, preferably less than 0.1 wt %, more preferably less than 0.01 wt %, of water, each based on the total weight of vildagliptin subjected to granulation or based on the total weight of acid addition salt of vildagliptin subjected to granulation; especially it is preferred to add no water during non-aqueous moisture activated granulation. The added water can be present either in free form or as a component of liquid(s).

Furthermore, it is preferred to add during aqueous moisture activated granulation less than 50 wt %, preferably between 1 and 50 wt %, preferably between 1 and 40 wt %, more preferably between 10 and 40 wt %, most preferably between 20 and 40 wt % of aqueous liquid, based on the total weight of vildagliptin subjected to granulation or based on the total weight of acid addition salt of vildagliptin subjected to granulation.

In particular, a moisture activated granulation, preferably a non-aqueous moisture activated granulation, may comprise contacting the mixture comprising vildagliptin or an acid addition salt thereof with a liquid. Preferably this liquid can be by sprayed onto the mixture comprising vildagliptin or an acid addition salt and/or can consist of or can comprise one or more of

propylene glycol, glycerol,

A granulate resulting of any of the processes described herein in more detail may be compressed in one or more comprimate(s), preferably in one or more tablet(s) or microtablet(s), or may be formulated in one or more pellet(s), which comprimate(s) or pellet(s) optionally can be coated and/or optionally filled into capsule(s) or sachet (s).

According to one embodiment, no drying step, preferably no drying step at a temperature of more than 25°C, is carried out following granulation. According to a further embodiment, no such drying step is carried out between granulation and compression.

According to another aspect, the present invention provides a granulate comprising vildagliptin or an acid addition salt thereof obtainable according to any of the processes according to the present invention.

According to yet another aspect, the present invention provides a pharmaceutical composition comprising or consisting of granulate(s) comprising vildagliptin or an acid addition salt thereof obtainable according to any of the processes according to the present invention. Furthermore, a pharmaceutical composition comprising or consisting of comprimate(s) obtainable according to any of the processes described in the present application is provided. Moreover, a pharmaceutical composition comprising or consisting of pellet(s) obtainable according to any of the processes described in the present application is provided. Preferably the pharmaceutical composition can be a solid dosage form, more preferably a solid oral dosage form in unit dose form, in particular a solid oral dosage form selected from the group consisting of tablets, in particular coated tablets, microtablets, and capsules filled with one or more of granulate(s), comprimate(s) and/or coated comprimate(s), and pellet(s) and/or coated pellet (s), which may be e.g obtained according to any of the processes described in the present application.

In one embodiment, the pharmaceutical composition may comprise at least one further active ingredient. Preferably, the at least one further active ingredient can be at least one active ingredient for the treatment of diabetes and/or at least one anti-obesity agent, more preferably at least one active ingredient selected from the group consisting of insulin, metformin, derivatives of sulphonyl urea, glitazone(s), rivastigmin, donepesil, galantamin, tegaserod, and sartans, in particular valsartan. When the pharmaceutical composition comprises at least one further active ingredient, a combination of granulation methods may be applied, i.e. each active ingredient may be present in a separate granulate, prepared by the same or a different granulation method.

A pharmaceutical composition may comprise the active ingredient vildagliptin or an acid addition salt thereof in an amount of 10 to 150 mg, preferably in an amount of 20 to 100 mg per unit dose form, preferably per tablet or per capsule.

Vildagliptin is sensitive to water and/or moisture and to oxidizing agents, such as peroxide, for examples, due to the presence of the OH group in vildagliptin's structure, so when including it into pharmaceutical formulations, care should be taken that the pharmaceutically acceptable excipient(s) used for its preparation do not contain a non-negligible quantity of them. Vildagliptin is further pH sensitive and is more stable at acidic pH values, e.g. at pH values from 1 to 7, particularly from 3 to 7.

The description likewise relates to the use of vildagliptin or of its pharmaceutically acceptable salts as pharmacologically active substances. In this connection, they can be used, preferably in the form of pharmaceutically acceptable preparations, in a method for the prophylactic and/or therapeutic treatment.

The invention likewise relates to solid pharmaceutical compositions which contain vildagliptin according to the invention or pharmaceutically acceptable salts thereof as active ingredients, and to processes for their preparation. The daily dose of the active ingredient depends on the age and the individual condition of the patient and also on the manner of administration.

The pharmaceutical preparations of vildagliptin contain, for example, from about 10 wt% to about 80 wt%, preferably from about 20 wt% to about 60 wt%, of the active ingredient, in particular of the active ingredient vildagliptin. Pharmaceutical preparations according to the invention for oral administration are, for example, those in unit dose forms, such as coated tablets or microtablets, tablets or microtablets, or capsules. These are prepared in a manner known per se, for example by means of conventional mixing, granulating, coating, dissolving or lyophilizing processes. Thus, pharmaceutical preparations for oral use can be obtained by combining the active ingredient with excipients, if desired granulating a mixture obtained, and processing the mixture or granules, if desired or necessary, after addition of suitable excipients to give tablets, microtablets or capsules, which can be optionally be coated.

Direct compression has some serious drawbacks due to the problems with content uniformity in case of low dose solid compositions and the need of using special types of excipients which are usually significantly more expensive which results in increased costs of production.

We have surprisingly found out that stable and bioavailable solid pharmaceutical compositions containing an effective amount of vildagliptin or its pharmaceutically acceptable salt can be produced on industrial scale by using conventional processes and equipment known from the state of the art and readily available in pharmaceutical industry, such as the production of solid compositions where the active ingredient vildagliptin or its salt in an amount of preferably 10 to 150 mg, more preferably 20 to 100 mg, is pregranulated by state of the art granulation techniques such as wet, dry or melt granulation. The obtained granulate is compressed in comprimates such as microtablets or tablets or formulated into pellets via extrusion and spheronisation. In one embodiment of the invention the obtained comprimates and pellets can optionally be filled into capsules or sachets or in another embodiment optionally coated with a film coating. The coated microtablets and pellets can also be filled into capsules or sachets. The film coating can be applied for one of the following reasons:

- To improve visual performance of the solid composition e.g colour,
- To ease the swallowing of the tablets, which is specially important for elderly patients
- To protect the active ingredient in the solid composition from the destructive influence of the environment such as moisture and oxygen from the surrounding air,
- To achieve delayed and/or prolonged release of the active ingredient from the solid composition,
- To prevent interaction with another active ingredient or an excipient formulated into the same solid composition.

Wet granulation can be performed by using granulation liquids based using an organic solvent selected from alcohols with 1 to 4 carbon atoms like absolute ethanol, concentrated ethanol (96 vol%), methanol, isopropanol, or mixtures thereof as solvents. Excipients selected from diluents, binders, antioxidants, pH modifiers, surfactants can optionally be dissolved, suspended, emulsified or dispersed into the granulation liquid which is sprayed onto the powder mixture comprising vildagliptin or its salt, a diluent and optionally other pharmaceutically acceptable excipients selected from a diluent, disintegrant, binder, antioxidant and/or pH modifier using state of the art granulators such as a high shear granulator, low shear granulator, or a fluid bed granulator. The obtained granules are dried to achieve loss on drying determined by halogen dryer Mettler HR73 (20 minutes at 85°C) of less than 4 wt% and optionally sieved through a sieve with sieve openings 1.00 mm. The granulation liquid used for wet granulation, in particular moisture-activated granulation, further in particular non-aqueous moisture-activated granulation is free of gelatine.

In a special embodiment of the invention vildagliptin or its salt can be dissolved or suspended in the granulation liquid composed of a solvent which is organic solvent selected from alcohols with 1 to 4 carbon atoms like absolute ethanol, concentrated ethanol (96 vol%), methanol, isopropanol, or mixtures thereof and other excipients as described above, which can be dissolve, suspended or emulsified in it. The obtained granulation liquid containing vildagliptin or its salt is sprayed onto the powder mixture of excipients selected from one or more diluent, disintegrant, binder, antioxidant and/or pH modifier using state of the art granulators such as high shear granulator, low shear granulator, or a fluid bed granulator. The obtained granulate is dried to achieve loss on drying determined by halogen dryer Mettler HR 73 (20 minutes at 85°C) of less than 4 wt% and optionally sieved through a sieve with sieve openings 1.00 mm.

As a special embodiment of wet granulation, a non-aqueous moisture activated granulation can be performed by using granulation liquids selected from liquid polyethylene glycol, propylene glycol, glycerol, liquid ethers of polyethylene glycol and alkyl alcohols, liquid paraffin, liquid fatty alcohols with at least 8 carbon atoms, liquid triglycerides or oils, liquid wax, liquid polyethoxylated fatty acids, liquid PEG glycerol fatty acid esters. Granulation liquid is sprayed onto the powder mixture comprising vildagliptin or its salt, a diluent and optionally other pharmaceutically acceptable excipients

selected from a diluent, disintegrant, binder, antioxidant and/or pH modifier using state of the art granulators such as a high shear granulator, low shear granulator, or a fluid bed granulator. The obtained granules are optionally dried and optionally sieved through a sieve with sieve openings of 1.00 mm.

In a special embodiment of the present invention vildagliptin or its salt can be formulated into spherical particle having average diameter in the range 300 to 2000 μm, preferably 500 to 1500 μm such as pellets. Pellets can be produced by the state of the art processes such as direct pelletisation in high shear mixer, rotor pelletisation, extrusion and spheronisation, layering of neutral cores in fluid bed coater with an active substance dispersed together with optional other excipients selected from diluent, binder, disintegrant, surfactant, antioxidant, pH modifier in an appropriate vehicle. The obtained pellets can optionally be further coated with a coating for achieving desired properties of final pellets such as stability, release rate and absorption of incorporated active ingredient as described above. In another embodiment the obtained pellets can be filled into capsules or compressed into tablets.

The granulate obtained by one of the processes described above can be compressed into round or oblong tablets having a maximal diameter in the range from 4 to 14 mm, preferably from 5 to 12 mm and most preferably from 6 to 10 mm; or into microtablets having diameter in the range from 1.5 to 3.5 mm, preferably from 2 to 3 mm; or can be filled into capsules or sachets. The hardness of the compressed tablets can be in the range between 50 to 150 N. The compressed tablets and microtablets can be optionally further coated by a film coating with a coating for achieving the desired properties of final solid composition such as stability, release rate and absorption of incorporated active ingredient as described above.

Solid compositions can be packed into primary packaging having decreased permeability for water vapour such as high density polyethylene (HDPE) containers with closure, such as polypropylene (PP) closure and with or without a desiccant; aluminium foil blisters or polychlorotrifluoroethylene (Aclar®) blisters or any other suitable water vapour low-permeable packaging such as blisters made of multilayer polymeric foil where different polymeric materials are combined into a single foil. Additionally inert gases such as nitrogen, argon or helium; or vacuum can be used during packaging to assure inert atmosphere around the dosage form, such as tablet or capsule, in the sealed primary packaging. Inert atmosphere according to present invention means that the concentration of oxygen in the atmosphere around the solid dosage form such as tablet containing vildagliptin or its salt packed in the primary packaging is less than 10 vol%, preferably less than 5 vol% and most preferably less than 2 vol%. The concentration of oxygen in the atmosphere surrounding the tablet can be determined by gas chromatography.

The diluent(s) can be selected from soluble carbohydrates such as lactose, e.g. lactose monohydrate, anhydrous lactose, spray dried and/or granulated lactose, sucrose, dextrin, sugar alcohols such as xylitol, mannitol, maltitol, isomalt, sorbitol, powdered cellulose, microcrystalline cellulose, silicified microcrystalline cellulose, low moisture starch, corn starch, pregelatinized starch, low moisture pregelatinized starch, calcium salts of phosphoric acid such as calcium hydrogen phosphate in anhydrous and hydrated form, calcium, sodium or potassium carbonate or hydrogen carbonate. Preferably, low moisture diluents are applied. Diluents used extragranularly can preferably have an average particle size in the range 80 to 600 μm, more preferably 100 to 500 μm, and diluents used intragranularlly can preferably have an average particle size in the range between 10 to 300 μm, more preferably 20 to 150 μm.

The binder for use in wet granulation is selected from cellulose derivatives which are hydroxymethylcellulose, hydroxyethylcellulose, hydroxypropylcellulose (hyprollose) and hydroxypropylmethylcellulose (hypromellose (reference example)) having a viscosity when measured in a 2 wt/vol% aqueous solution at 20°C in the range between 2 to 20 mPas, preferably from 3 to 15 mPas measured according to the USP/NF method, and mixtures thereof. Hydroxymethylcellulose and hydroxyethylcellulose are particularly preferred.

The term "disintegrant" refers to the group of compounds comprising a NVP water-swellable polymer, crospovidone, starch, pregelatinized starch, sodium starch glycolate, hydroxypropyl starch, microcrystalline cellulose, carboxymethylcellulose sodium and calcium, cross-linked carboxymethylcellulose sodium, cross-linked carboxymethylcellulose calcium, polacrilin potassium, low-substituted hydroxypropylcellulose, sodium and calcium alginate, docusate sodium, methylcellulose, agar, guar gum, chitosan and alginic acid. Low-substituted hydroxypropycellulose is preferred. A NVP water-swellable polymer is an insoluble, swellable homo- or heteropolymer containing N-vinylpyrrolidone, e.g. N-vinyl-2-pyrrolidone.

Surfactants can in general be selected from nonionic, ampholitic or ionic surfactants having an HLB value (HLB value denotes the Hydrophilic-Lipophilic Balance of a surfactant and is a measure of the degree to which it is hydrophilic or lipophilic, determined by calculating values for the different regions of the molecule, as described by Griffin or by Davies) of more than 8, preferably of more than 10.

Nonionic surfactants can in particular be selected from polyoxyethylated glycol monoethers, cetomacrogol, sorbitan esters (Spans) and polysorbates (Tweens), polyoxyethylene-polyoxypropylene copolymers such as poloxameres, sugar esters with fatty acids with 10 to 22 C atoms.

Ionic surfactants can in particular be selected from the group of anionic surfactants such as organic sulphonates ($RSO_3^-$), sulphates ($ROSO_3^-$) e.g. sodium lauryl sulphate $CH_3(CH_2)_{11}SO_4^-Na^+$, potassium laurate $CH_3(CH_2)_{10}COO^-K^+$ or cationic surfactants selected from the group consisting of organic quaternary ammonium halides, $R_4N^+Cl^-$, cetrimide,

a mixture consisting of tetradecyl (about 68%), dodecyl (about 22%), and hexadecyltrimethylammonium bromides (about 7%), as well as benzalkonium chloride, a mixture of alkylbenzyldimethylammonium chlorides of the general formula $[C_6H_5CH_2N^+(CH_3)_2R]Cl^-$, where R represents a mixture of alkyls from $C_8H_{17}$ to $C_{18}H_{37}$.

Ampholytic surfactants are selected from sulfobetaines $RN^+(CH_3)_2CH_2CH_2SO_3^-$), or betains N-Dodecyl-N,N-Dimethylbetaine, $C_{12}H_{25}N^+(CH_3)_2CH_2COO^-$.

The antioxidant(s) can be selected from the phenole functional group containing substances such as butyl hydroxyanizole (BHA), butylhydroxytoluene (BHT), rutin, quercetin, coffee acid, ascorbic acid or its metal salts, e.g. sodium ascorbate, sorbic acid, citric acid etc.

The pH modifier is used to adjust the pH of the microenvironment of vildagliptin particles to a pH value in the range between 3 to 9 and can be selected from amino acids, e.g. phenylalanine, organic carboxylic acids such as fumaric, citric, tartaric, benzoyltartaric, 4-methylbenzoyltartaric, galic, ferrulic, malonic, glutaric, maleilic, malic, adipic, succinic acid; from soluble and low soluble metal salts of anorganic and/or organic carboxylic acids such as the sodium, potassium, magnesium, calcium, aluminium salts of phosphoric, carbonic, citric, and tartaric acid such as sodium hydrogen carbonate, sodium carbonate, magnesium carbonate, potassium carbonate, disodium hydrogen phosphate, trisodium phosphate, sodium citrate, meglumine, metal salts of amino acids, and glucosamin.

The adsorbent can be selected from anorganic materials having a specific area of more than 25 $m^2/g$ preferably of more than $50m^2/g$, such as magnesium aluminium silicate obtainable as Neusilin, colloidal silica sold as Aerosil 200, bentonite, or zeolite.

The lubricant may be chosen from the group comprised of metal salts of fatty acids with 12 to 20 carbon atoms such as magnesium, calcium, aluminium or zinc stearate, magnesium palmitate and magnesium oleate; hydrogenated vegetable oil, hydrogenated castor oil, talc, beads wax or spermaceti, boric acid, sodium stearyl fumarate, macrogols and mixtures thereof. Stearic acid, magnesium stearate and hydrogenated vegetable oil are particularly preferred. The solid composition comprises from 0.1 to 10 wt.-%, particularly from 0.25 to 5 wt.-%, more preferably from 0.5 to 2 wt.-% of a lubricant. Preferably, the magnesium salt of stearic acid is used.

The glidant can be selected from colloidal silica (Aerosil 200 or Aerosil R972) and magnesium trisilicate.

The pigment can be selected from titanium dioxide, iron oxides, or lakes.

The antitacking agent is selected from talc, glycerolmonostearate, or stearic acid.

The plasticizer for hot melt extrusion polymers can be selected from dibutilsebacate, polyethylenglycole with average molecular weight 400 to 10000, preferably 1500 to 8000 g/mol, or triethylcitrate,

Hot melt extrusion polymers can be selected from polymers having glas transition or softening point below 190°C, preferably below 170°C such as but not limited to povidone with K value 12 to 90, copovidone, hyprolose, hypromellose, methylcellulose, polymethacrylate derivatives such as Eudragit® E or Eudradit S.

Solid composition containing vildagliptin or its salts can optionally contain at least one further active ingredient, e.g. for the treatment of diabetes or an anti-obesity agent, insulin, metformin, derivaties of sulphonyl urea, glitazone(s), rivastigmin, donepesil, galantamin, tegaserod, or a sartan, in particular valsartan.

In one embodiment vildagliptin and the other active ingredient(s), preferably metformin, are mixed with excipients homogenously and compressed into tablets via a granulation step, where wet granulation process can be used as described above. In another embodiment different active ingredients are incorporated into different granulates which can be mixed and compressed into tablets or the prepared granulates can by compresses stepwise into multilayer tablets such as a bilayer tablet in case of combining two incompatible active ingredients. In still another embodiment of the present invention spherical particles of different sub-batches each containing only one active substance are prepared as described above are mixed with excipients and filled into capsules or compressed into tablets.

Particle size was measured using a Malvern-Mastersizer 2000 equipped with Hydro S dispersion unit. Vegetable oil, in particular corn oil, was used as the dispersion medium, no sonnication was applied. Absorption value was set to 1 (A=1).

The term "average particle size" as used herein refers to the volume mean diameter of particles.

The present invention can be illustrated in one of its embodiments by the following non-limiting example.

## EXAMPLES

### Example 1A

<u>Preparation of (S)-1-(2-chloroacetyl)pyrrolidin-2-carboxamide</u>

A 25 mL dry flask, equipped with a magnetical stirrer and inertized with Ar atmosphere, is charged with 0.46 g (4 mmol) L-prolinamide, 300 mL of water and 10 mL tetrahydrofuran. First on an ice bath and later at a temperature below 35°C .54 g (4.8 mmol) chloroacetyl chloride is added. The reaction mixture is stirred at room temperature for 1.5 hours. After completion of the reaction, 0.55 g of potassium carbonate (4 mmol) are added. The volatile components

are partly evaporated and the residue is dissolved in 30 mL of dichloromethane. The organic phase is washed 3-times with 10 mL of water, and 10 mL of brine. The organic phase is dried over anhydrous $Na_2SO_4$, filtered and the solvent is evaporated *in vacuo.*

**Example 1B**

Preparation of (S)-1-(2-chloroacetyl)pyrrolidin-2-carboxamide

A 25 mL dry flask, equipped with a magnetical stirrer and inertized with Ar atmosphere, is charged with 0.46 g (4 mmol) L-prolinamide, 300 mL of water and 10 mL tetrahydrofuran. First on an ice bath and later at a temperature below 35°C .54 g (4.8 mmol) chloroacetyl chloride is added. The reaction mixture is stirred at room temperature for 1.5 hours. After completion of the reaction, 0.55 g of potassium carbonate (4 mmol) are added. The volatile components are partly evaporated and the residue is dissolved in 30 mL of dichloromethane. The organic phase is washed 3-times with 10 mL of water, and 10 mL of brine. The organic phase is dried over anhydrous $Na_2SO_4$, filtered and the solvent is evaporated *in vacuo.*

**Example 2A**

Preparation of (S)-1-(2-chloroacetyl)pyrrolidin-2-carbonitrile

A 500 mL reactor, equipped with a mechanical stirrer and inertized with Ar atmosphere, is charged with 62.97 g (455.6 mmol, 4 equiv.) of $K_2CO_s$, 13.00 g of L-prolinamide (113.9 mmol, 1 equiv.), 50 mL of anhydrous dimethylacetamide and 100 mL freshly distilled tetrahydrofuran. At a temperature below 35°C 10.42 mL (131 mmol, 1.15 equiv.) chloroacetyl chloride is added,. The reaction mixture is stirred for 2 hours. After completion of the reaction, $K_2CO_3$ is filtered and the filtrate is taken to a dry and inertized 500 mL reactor. 23.75 mL (170.85 mmol, 1.5 equiv.) of trifluoroacetic anhydride are added and the reaction mixture is left stirring for another hour. After completion of the dehydration reaction the volatile components are evaporated and the residue is dissolved in 300 mL of dichloromethane. The organic phase is washed 3-times with 100 mL of a saturated aqueous solution of sodium hydrogencarbonate and 100 mL of brine. The organic phase is dried over anhydrous $Na_2SO_4$, filtered and the solvent is evaporated *in vacuo.* 20 g of an oily product is obtained, which is further purified by crystallizing it from isopropyl alcohol (65 mL) to yield 14.5 g of white crystals (yield 74%, chromatographic purity 99.9%, content of R-enantiomer 0.04 %, melting point 54- 57°C).

**Example 2B**

Preparation of (S)-1-(2-chloroacetyl)pyrrolidin-2-carbonitrile

A 25 mL dry flask, equipped with a magnetical stirrer and inertized with Ar atmosphere, is charged with 1 g (8.76 mmol, 1 equiv.) L-prolinamide, 4 mL of anhydrous dimethylacetamide, and 5 mL freshly distilled tetrahydrofuran. First on an ice bath and later at a temperature below 35°C 0.80 mL (10.07 mmol, 1.15 equiv.) chloroacetyl chloride is added. A white precipitate is formed which dissolves after the addition of 1 mL of dimethylacetamide. The reaction mixture is stirred at room temperature for 1.5 hours.

After completion of the reaction, 1.83 mL (13.14 mmol, 1.5 equiv.) of trifluoroacetic anhydride are added on ice bath. The reaction mixture is allowed to warm to room temperature and left stirring for another hour. After completion of the dehydration reaction the volatile components are partly evaporated and the residue is dissolved in 30 mL of dichloromethane. The organic phase is washed 3-times with 10 mL of water, 3-times with a saturated aqueous solution of sodium hydrogencarbonate, and 10 mL of brine. The organic phase is dried over anhydrous $Na_2SO_4$, filtered and the solvent is evaporated *in vacuo.* 1.29 g of an oily product is obtained, which solidifies eventually. The chemical yield of the reaction was 85 %. The crude product is further purified by crystallizing it from isopropyl alcohol (4 mL). The yield of the crystallization is 74% and the complete yield of the process is 63 %. The chromatographic purity of the product obtained is 98.4% and the content of the R-isomer is 0.09%.

**Example 3**

Preparation of vildagliptin (I)

A 500 mL reactor, inertized with Ar, is charged with 15.60 g (93.3 mmol, 1.15 equiv.) of 3-amino-1-adamantole, 22.42 g (162.2 mmol, 2 equiv.) of $K_2CO_3$, and 3.70 g (16.2 mmol, 0.2 equiv.) of benzyl triethyl ammonium chloride. 150 mL of acetonitrile are added and the reaction mixture is vigorously stirred. One half of a solution prepared by dissolving

14.00g (81.1 mmol, 1 equiv.) of (S)-1-(2-chloroacetyl)pyrrolidin-2-carbo-nitrile in 50 mL of acetonitrile is slowly added. The reaction mixture is stirred at 45°C and after 2 hours, one 1/4 of the solution of (S)-1-(2-chloroacetyl)pyrrolidin-2-carbo-nitrile as above is added. After another 2 hours of stirring, the last ¼ of the solution of (S)-1-(2-chloroacetyl)pyrrolidin-2-carbo-nitrile is added. The reaction mixture is stirred at 45°C for 12 hours, the solid phase is filtered off and the volatile components of the liquid phase are evaporated under reduced pressure. The residue is dissolved in 200 mL of dichloromethane and washed with 100 mL of water. The aqueous phase is washed twice with 100 mL of dichloromethane. The combined organic phases are dried over anhydrous Na2SO4, filtered and the solvent is evaporated under reduced pressure. The crude vildaglitpin obtained is re-crystallized from 2-butanone (70 mL). 10.2 g of white crystals with a melting point of 149-150°C and a purity of 99.8% as determined by HPLC are obtained (41% yield).

**Example 4**

Preparation of hydrochloride salt of (S)-1-(2-chloroacetyl)pyrrolidin-2-carbonitrile

2.27 g (13.18 mmol) of (S)-1-(2-chloroacetyl)pyrrolidin-2-carbonitrile is dissolved in 24 mL of isopropyl alcohol at 70°c. Then 1.36 g hydrochloric acid (37% (w/w) solution in water) are added dropwise over ca. 5-10 minutes. The solution is allowed to cool to room temperature (between 20 and 25°C). A a constant dropping rate, 5 mL of tert-butyl methyl ether are added over 10 minutes. The resulting suspension was stirred at room temperature for 3 hours and then filtered. The crystals were washed with 10 mL isopropyl alcohol and dried at 60°C/15 mbar for 5 hours.

**Example 5**

Preparation of hydrochloride salt of (S)-1-(2-chloroacetyl)pyrrolidin-2-carboxamide

2.51 g (13.18 mmol) of (S)-1-(2-chloroacetyl)pyrrolidin-2-carboxamide is dissolved in 24 mL of isopropyl alcohol at 70°c. Then 1.36 g hydrochloric acid (37% (w/w) solution in water) are added dropwise over ca. 5-10 minutes. The solution is allowed to cool to room temperature (between 20 and 25°C). A a constant dropping rate, 5 mL of tert-butyl methyl ether are added over 10 minutes. The resulting suspension was stirred at room temperature for 3 hours and then filtered. The crystals were washed with 10 mL isopropyl alcohol and dried at 60°C/15 mbar for 5 hours.

**PREPARATION OF SOLID ORAL DOSAGE FORMS COMPRISING VILDAGLIPTIN**

**Examples 6-15:**

Tablets were prepared according to the wet granulation process as follows.

*1) Preparation of granulates*

The respective solid components shown in Table 1 below were homogenized in a high shear (HS) mixer or in a fluid bed granulator (FBG), and ethanol was sprayed over this mixture. Alternatively, a part or a whole amount of binder can optionally be dissolved into the granulation liquid which is sprayed onto the powder mixture shown in Table 1 below. The obtained granulate was dried in a drying chamber (DC) or a fluid-bed dryer (FBD) at a specified temperature. The nature of the solid components and of the ethanol, mixing/granulating device, drying device and drying temperature are shown in Table 1.

Granulates 6a, 8a, 11a are reference examples.

**Table 1.**

| | Granulate | | | | |
|---|---|---|---|---|---|
| | **6a** | **7a** | **8a** | **9a** | **10a** |
| Components (mg) | | | | | |
| Vildagliptin | 50.00 | 50.00 | 50.00 | 50.00 | 50.00 |
| Microcrystalline cellulose[1)] | 50.00 | - | - | - | 35.00 |
| Mannitol | - | 35.00 | - | - | - |
| Lactose monohydrate | - | - | 50.00 | - | 15.00 |
| LH-B1[2)] | - | - | - | 50.00 | - |
| Povidone | 6.00 | - | - | - | - |
| Klucel EF[3)] | - | 5.00 | - | - | 6.00 |
| Hypromellose | - | - | 6.00 | - | - |
| LH-21[4)] | - | 10.00- | - | 6.00 | - |
| Primojel[5)] | 10.00 | | - | - | - |
| Crospovidone | - | | - | - | - |
| Ac-Di-Sol[6)] | - | - | 5.00 | - | - |
| Starch 1500[7)] | - | - | - | 20.00 | - |
| Starch | - | - | - | - | 20.00 |
| Ethanol[8)] | q.s. | q.s. | q.s. | q.s. | q.s. |
| Mixing/granulating device | HS | HS | HS | HS | HS |
| Drying method | DC | DC | DC | DC | DC |
| Drying temperature (°C) | 50-55 | 45-60 | 50-55 | 50-60 | 55-65 |

**Table 1 (continued).**

| | Granulate | | | | |
|---|---|---|---|---|---|
| | **11a** | **12a** | **13a** | **14a** | **15a** |
| Components (mg) | | | | | |
| Vildagliptin | 50.00 | 50.00 | 50.00 | 50.00 | 50.00 |
| Microcrystalline cellulose[1)] | 15.00 | 25.00 | 25.00 | - | - |
| Mannitol | - | - | - | 25.00 | 35.00 |
| Lactose monohydrate | 35.00 | 25.00 | - | 25.00 | - |
| LH-B1[2)] | - | - | 25.00 | - | |
| Povidone | 6.00 | - | - | - | - |
| Klucel EF[3)] | - | 6.00 | - | - | 5.00 |
| Hypromellose | - | - | 6.00 | - | - |
| LH-21[4)] | - | - | - | 6.00 | 10.00 |
| Primojel[5)] | 10.00 | - | - | - | - |
| Crospovidone | - | 20.00 | - | - | - |
| Ac-Di-Sol[6)] | - | - | 5.00 | - | - |
| Starch 1500[7)] | - | - | - | 20.00 | - |
| Starch | - | - | - | - | |
| Ethanol[8)] | q.s. | q.s. | q.s. | q.s. | q.s. |
| Mixing/granulating device | FBG | FBG | FBG | FBG | FBG |
| Drying method | FBD | FBD | FBD | FBD | FBD |
| Drying temperature (°C) | 35-55 | 35-55 | 35-55 | 30-50 | 35-55 |

[1)] Microcrystalline cellulose: commercially available as Avicel PH101, Avicel PH102, Avicel PH112, Avicel PH200, Avicel PH200 LM, Vivapur 12, Vivapur 14 or Vivapur 200

[2] LH-B1: commercially available low-substituted hydroxypropylcellulose

[3] Klucel EF: commercially available hydroxypropylcellulose

[4] LH-21: commercially available low-substituted hydroxypropylcellulose

[5] Primojel: commercially available sodium starch glycollate

[6] Ac-Di-Sol: commerically available croscarmellose sodium

[7] Starch 1500: commercially available pregelatinised starch

[8] ethanol can be selected from absolute ethanol or diluted ethanol with water

*2) Preparation of compression mixtures*

The components shown in Table 2 below were added to the respective granulates **(6a)-(15a)** obtained above and mixed in a high-shear mixer (HSM) or container mixer (CM) to obtain compression mixture(s). The nature of the added components as well as the type of the mixer are shown in Table 2.

**Table 2.**

| | Compression mixture | | | | |
|---|---|---|---|---|---|
| | **6b** | **7b** | **8b** | **9b** | **10b** |
| Granulate | **6a** | **7a** | **8a** | **9a** | **10a** |
| Components (mg) | | | | | |
| Microcrystalline cellulose[1] | 71.00 | -47.00 | - | - | |
| Mannitol[2] | - | | - | - | -40.00 |

| | | | | | |
|---|---|---|---|---|---|
| Lactose monohydrate[3)] | - | - | 76.00 | - | 21.00 |
| LH-B1 | 10.00 | - | - | 71.00 | - |
| LH-11[4)] | - | | - | - | - |
| LH-21 | - | - | 10.00 | - | - |
| LH-22[5)] | - | - | - | - | - |
| Primojel[6)] | - | 8.00- | - | - | 10.00 |
| Crospovidone | - | - | - | - | - |
| Ac-Di-Sol | - | - | - | - | - |
| Starch 1500 | - | - | - | - | - |
| Aerosil R972 | | 2.25 | | | |
| Magnesium stearate | 3.00 | 2.75 | 3.00 | 3.00 | 3.00 |
| Mixing device | HSM | CM | HSM | CM | HSM |

**Table 2 (continued).**

| | Compression mixture | | | | |
|---|---|---|---|---|---|
| | **11b** | **12b** | **13b** | **14b** | **15b** |
| Granulate | **11a** | **12a** | **13a** | **14a** | **15a** |
| Components (mg) | | | | | |
| Microcrystalline cellulose[1)] | 61.00 | - | - | - | 47.00 |
| Mannitol[2)] | - | - | 46.00 | 61.00 | |
| Lactose monohydrate[3)] | - | - | - | - | - |
| LH-B1 | - | 61.00 | - | - | - |
| LH-11[4)] | - | - | 20.00 | - | |
| LH-21 | - | - | - | - | - |
| LH-22[5)] | - | - | - | - | - |
| Primojel[6)] | - | - | - | 10.00 | 8.00- |
| Crospovidone | 20.00 | - | - | - | - |

| | | | | | |
|---|---|---|---|---|---|
| Ac-Di-Sol | - | 10.00 | - | - | - |
| Starch 1500 | - | - | 20.00 | - | - |
| Aerosil R972[7)] | | | | | 2.25 |
| Magnesium stearate | 3.00 | 3.00 | 3.00 | 3.00 | 2.75 |
| Mixing device | CM | HSM | CM | HSM | CM |

[1)] Microcrystalline cellulose: commercially available as Avicel PH102, Avicel PH112, Avicel PH200 or Avicel PH200 LM

[2)] Mannitol: commercially available for direct compression, e.g. Partec

[3)] Lactose monohydrate: commercially available for direct compression, e.g. Tablettose, Pharmatose, SuperTab, Lactochem, CapsuLac, GranuLac, PrismaLac, SacheLac, SorboLac, SpheroLac, Wyndale

[4)] LH-11: commercially available low-substituted hydroxypropylcellulose

[5)] LH-22: commercially available low-substituted hydroxypropylcellulose

[6)] Primojel: commercially available sodium starch glycollate

[7)] Aerosil R972: commercially available hydrophobic colloidal silicon dioxide

*3) Preparation of tablets*

The compression mixtures **(6b)-(15b)** were compressed into round or oval tablets with a theoretical weight of 150-200 mg.

**Examples 16-20**

Tablets were prepared according to the wet granulation process as follows by dissolving vildagliptin in a solvent.

*1) Preparation of granulates*

The respective components shown in Table 3 below were homogenized in fluid bed granulator, and a dispersion of vildagliptin in liquid solvent was sprayed over this mixture. The obtained granulate was dried in a fluid-bed dryer at a specified temperature.

The nature of the solid components and of the solvent, mixing/granulating device, drying device and drying temperature are shown in Table 3.

Granulates 16a, 18a are reference examples.

**Table 3.**

| | Granulate | | | | |
|---|---|---|---|---|---|
| | **16a** | **17a** | **18a** | **19a** | **20a** |
| Components (mg) | | | | | |
| Vildagliptin | 50.00 | 50.00 | 50.00 | 50.00 | 50.00 |
| Microcrystalline cellulose[1)] | 50.00 | - | - | - | 35.00 |
| Mannitol | - | 50.00 | - | - | - |
| Lactose monohydrate | - | - | 50.00 | - | 15.00 |
| LH-B1[2)] | - | - | - | 50.00 | - |
| Povidone | 6.00 | - | - | - | - |
| Klucel EF[3)] | - | 6.00 | - | - | 6.00 |
| Hypromellose | - | - | 6.00 | - | - |
| LH-21[4)] | - | - | - | 6.00 | - |
| Primojel[3)] | 10.00 | - | - | - | - |
| Crospovidone | - | 20.00 | - | - | - |
| Ac-Di-Sol[6)] | - | - | 5.00 | - | - |
| Starch 1500[7)] | - | - | - | 20.00 | - |
| Starch | - | - | - | - | 20.00 |
| Ethanol[8)] | q.s. | - | - | q.s. | - |
| Methanol | - | q.s. | - | - | q.s. |
| Isopropanol | - | - | q.s. | - | - |
| Purified water | - | - | - | q.s. | q.s. |
| Drying temperature (°C) | 50-55 | 55 | 50-55 | 50-60 | 55-65 |

[1)] Microcrystalline cellulose: commercially available as Avicel PH101, Avicel PH102 or Avicel PH200
[2)] LH-B1: commercially available low-substituted hydroxypropylcellulose
[3)] Klucel EF: commercially available hydroxypropylcellulose
[4)] LH-21: commercially available low-substituted hydroxypropylcellulose
[5)] Primojel: commercially available sodium starch glycollate
[6)] Ac-Di-Sol: commerically available croscarmellose sodium
[7)] Starch 1500: commercially available pregelatinised starch
[8)] ethanol can be selected from absolute ethanol or diluted ethanol with water

*2) Preparation of compression mixtures*

The components shown in Table 4 below were added to the respective granulates **(16a)-(20a)** obtained above and mixed in a high-shear mixer (HSM) or container mixer (CM) to obtain compression mixture. The nature of the added components as well as the type of the mixer are shown in Table 4.

**Table 4.**

| | Compression mixture | | | | |
|---|---|---|---|---|---|
| | **16b** | **17b** | **18b** | **19b** | **20b** |
| Granulate | **16a** | **17a** | **18a** | **19a** | **20a** |
| Components (mg) | | | | | |
| Microcrystalline cellulose | 71.00 | - | - | - | 40.00 |

(continued)

| Components (mg) | | | | | |
|---|---|---|---|---|---|
| Mannitol | - | 61.00 | - | - | - |
| Lactose monohydrate | - | - | 76.00 | - | 21.00 |
| LH-B1 | 10.00 | - | - | 71.00 | - |
| LH-11 | - | 10.00 | - | - | - |
| LH-21 | - | - | 10.00 | - | - |
| Primojel | - | - | - | - | 10.00 |
| Magnesium stearate | 3.00 | 3.00 | 3.00 | 3.00 | 3.00 |
| Mixing device | HSM | CM | HSM | CM | HSM |

*3.) Preparation of tablets*

The compression mixtures **(16b)-(20b)** were compressed into round or oval tablets with a theoretical weight of 200 mg.

**Examples 21-25** (Reference)

Tablets were prepared according to the dry granulation process, i.e. in the absence of water, as follows.

*1) Preparation of granulates*

The respective solid components shown in Table 5 below were homogenized in a container mixer. The homogenized material was compacted using a roller compactor by applying a compaction force and a roller speed up to 7 rpm. The compacted material was sieved again to prepare sieved granulate.

The nature of the solid components are shown in Table 5.

**Table 5. Composition of granulate.**

| | Granulate | | | | |
|---|---|---|---|---|---|
| | **21a** | **22a** | **23a** | **24a** | **25a** |
| Components (mg) | | | | | |
| Vildagliptin | 50.00 | 50.00 | 50.00 | 50.00 | 50.00 |
| Microcrystalline cellulose | 100.00 | - | - | - | 50.00 |
| Mannitol | - | 100.00 | - | - | - |
| Lactose monohydrate | - | - | 100.00 | - | - |
| LH-B1 | - | - | - | 100.00 | 50.00 |
| LH-11 | 10.00 | - | - | - | - |
| Primojel | - | 10.00 | - | - | - |
| Crospovidone | - | - | 20.00 | - | - |
| Aerosil | 10.00 | 10.00 | 10.00 | 10.00 | 10.00 |
| Magnesium stearate | 1.50 | 1.50 | 1.50 | 1.50 | 1.50 |

*2) Preparation of compression mixtures*

The components shown in Table 6 below were added to the respective granulates **(21a)-(25a)** obtained above and mixed in a high-shear mixer (HSM) or conic mixer (CM) to obtain compression mixture. The nature of the added components as well as the type of the mixer are shown in Table 6.

**Table 6. Composition of compression mixture.**

| | Compression mixture | | | | |
|---|---|---|---|---|---|
| | **21b** | **22b** | **23b** | **24b** | **25b** |
| Granulate | **21a** | **22a** | **23a** | **24a** | **25a** |
| Components (mg) | | | | | |
| Microcrystalline cellulose | 26.00 | - | - | - | - |
| LH-B1 | - | 27.00 | - | - | - |
| LH-11 | - | - | 16.00 | - | - |
| Primojel | - | - | - | 37.00 | - |
| Crospovidone | - | - | - | - | 36.50 |
| Aerosil | 1.00 | - | 1.00 | - | 1.00 |
| Magnesium stearate | 1.50 | 1.50 | 1.50 | 1.50 | 1.50 |

*3) Preparation of tablets*

The compression mixtures **(21b)-(25b)** were compressed into round or oval tablets with a theoretical weight of 200 mg.

**Examples 26-30: hot melt granulation** (Reference)

**Table 7: Composition of granulate obtained by hot melt granulation**

| | Composition of granulate (mg/unit) | | | | |
|---|---|---|---|---|---|
| | **26a** | **27a** | **28a** | **29a** | **30a** |
| Components (mg) | | | | | |
| Vildagliptin | 50.00 | 50.00 | 50.00 | 50.00 | 50.00 |
| Low-substituted hydroxypropylcellulose | 10.00 | 10.00 | 10.00 | 15.00 | 10.00 |
| Gelucire 50/13 | 10.00 | - | - | - | - |
| Polyethylenglycol 6000 | - | 9.00 | - | 7.50 | 15.00 |
| Glycerolmono-stearate | - | - | 7.50 | - | - |
| AerosilR972 | 1.00 | 2.00 | 2.00 | 2.50 | |
| Lactose, anhydrous | 25.00 | 24.00 | - | - | 20.00 |
| Mannitol | - | - | 27.50 | 20.00 | - |
| Sodium starch glycolate | - | 5.00 | - | 5.00 | - |
| Croscarmellose sodium | 4.00 | - | 5.00 | - | 5.00 |
| Weight of intragranular phase | 100.00 | 100.00 | 100.00 | 100.00 | 100.00 |

Granulation procedure for Examples **(26a)-(29a):** all components are mixed in the laboratory high shear mixer Collette Gral 10 equipped with double jacked product bowl and heated to approx. melting temperature of the binder while mixing. The mixing continued until granulate is obtained. The obtained mass was cooled to approx. 20°C and sieved with oscilating sieve.

Granulation process for Example **(30a)**: all components except polyethyleneglycol 6000 are mixed in the laboratory high share mixer Collette Gral 10 equipped with double jacked product bowl. Melted binder (polyethyleneglycol 6000) was sprayed onto the powder mixture while mixing. The obtained granulate was cooled to approx. 20°C and

sieved with oscilating sieve.

**Table 8: Composition of tablets with granulate from hot melt granulation**

| | Compression mixture | | | | |
|---|---|---|---|---|---|
| | **26b** | **27b** | **28b** | **29b** | **30b** |
| Granulate | **26a** | **27a** | **28a** | **29a** | **30a** |
| Components (mg) | | | | | |
| granulate | 100.00 | 100.00 | 100.00 | 100.00 | 100.00 |
| Lactose for direct compression | 34.00 | - | 32.50 | - | |
| Mannitol for direct compression | - | - | - | 48.00 | - |
| Microcrystalline cellulose | 34.00 | 48.00 | - | | 45.50 |
| Sodium starch glycolate | 4.00 | - | - | 8.00 | 12.00 |
| Croscarmellose sodium | 4.00 | 8.00 | 10.00 | - | - |
| Aerosil R972 | 1.50 | 1.50 | 1.00 | 1.50 | - |
| Magnesium stearate | 2.50 | 2.50 | 1.50 | 2.50 | 2.50 |
| Total mass of tbl. | 180.00 | 160.00 | 145.00 | 160.00 | 160.00 |

**Example 27c:** Coating of tablets: Tablets from example 27 b were coated with Kollicoat Protect dispersion containing 12wt% Kollicoat Protect, 5wt% talc, 3wt% titanium dioxide and 80wt% water. The average thickness of the coating was 17 $\mu$m (measured from SEM micrograph of tablet crosssection).

**Example 27d:** Microtablets: Granulate from example 27a was compressed into microtablets having diameter of 2.2 mm. Each microtablet contain 5 mg of vildagliptin. The obtained microtablets were coated with with Eudragit EPO dispersion containing 7.1wt% Eudragit E PO, 1wt% stearic acid, 0.7wt% sodium lauryl sulphate, 3.6wt% talc, 1.8wt% Iron oxide red, 1.8% titanium dioxide and 84wt% purified water. The coating dispersion was applied nto the microtablets in Manesty XL coating drum in quantity corresponding to 4.2 mg of polymer/$cm^2$ of the microtablets.

**Examples 31-34: Moisture activated granulation**

**Table 9: Composition of granulate** (Reference)

| | **31a** | **32a** | **33a** | **34a** |
|---|---|---|---|---|
| Components (mg) | | | | |
| Vildagliptin | 50.00 | 50.00 | 50.00 | 50.00 |
| LH-B1 | 7.50 | 10.00 | 7.50 | 10.00 |
| Mannitol | 16.00 | - | 16.00 | - |
| Xylitol | - | 15.50 | - | 15.50 |
| Aerosil R 972 | 1.50 | 2.50 | 1.50 | 2.50 |
| Povidone K30 | 10.00 | 12.00 | - | - |
| Klucel EF | - | - | 10.00 | 12.00 |
| Purified water | 17.00 | q.s. | q.s | q.s |
| Weight of intragranular phase | 85.00 | 90.00 | 85.00 | 90.00 |

Granulation process: all solid components are sieved and transferred to product bowl of laboratory high shear mixer Collette Gral 10. Purified water was slowly sprayed over the moving powder mixture through a nozzle. Purified water used for granulation was used in ratio to total weight of dry components of 1 to 5. The obtained wet granulate was

dried in fluid bed dryer.

**Table 10: Composition of tablets**

| | Compression mixture | | | |
|---|---|---|---|---|
| | **31b/1** | **30b/2** | **32b/1** | **32b/2** |
| Granulate | **31a** | **31a** | **32a** | **32a** |
| Components (mg) | | | | |
| Granulate | 85.00 | 85.00 | 90.00 | 90.00 |
| LH-B1 | 37.00 | 27.00 | - | 35.00 |
| LH-11 | - | - | 15.00 | - |
| Granular lactose Super-Tab GR | 35.50 | - | 37.50 | 73.50 |
| Pearlitol 360 | - | 40.50 | - | - |
| Microcrystalline cellulose | - | 15.00 | 25.00 | - |
| Aerosil | 1.00 | 1.00 | 1.00 | - |
| Magnesium stearate | 1.50 | 1.50 | 1.50 | 1.50 |
| Total mass of tbl. | 160.00 | 170.00 | 170.00 | 200.00 |

**Table 10 (continued).**

| | Compression mixture | | | |
|---|---|---|---|---|
| | **33b/1** | **33b/2** | **34b/1** | **34b/2** |
| Granulate | **33a** | **33a** | **34a** | **34a** |
| Components (mg) | | | | |
| Granulate | 85.00 | 85.00 | 90.00 | 90.00 |
| LH-B1 | 37.00 | 27.00 | - | 35.00 |
| LH-11 | - | - | 15.00 | - |
| Granular lactose Super-Tab GR | 35.50 | - | 37.50 | 73.50 |
| Pearlitol 360 | - | 40.50 | - | - |
| Microcrystalline cellulose | - | 15.00 | 25.00 | - |
| Aerosil | 1.00 | 1.00 | 1.00 | - |
| Magnesium stearate | 1.50 | 1.50 | 1.50 | 1.50 |
| Total mass of tbl. | 160.00 | 170.00 | 170.00 | 200.00 |

All the components except Aerosil and magnesium stearate are mixed in laboratory biconical mixer for 10 minutes than first Aerosil and than magnesium stearate are added and mixed for another 1 minute. The obtained compression mixture was compressed on rotary tablet press into tablets.

**Examples 35-39:**

Tablets were prepared according to the wet granulation process as follows.

*1) Preparation of granulates*

The respective solid components shown in Table 11 below were homogenized in a high shear (HS) mixer or in a fluid bed granulator (FBG), and purified water was sprayed over this mixture. The obtained granulate was dried in a drying chamber (DC) or a fluid-bed dryer (FBD) at a specified temperature.

The nature of the solid components, mixing/granulating device, drying device and drying temperature are shown in Table 11.

**Table 11.**

| | Granulate (Reference) | | | | |
|---|---|---|---|---|---|
| | **35a** | **36a** | **37a** | **38a** | **39a** |
| Components (mg) | | | | | |
| Vildagliptin | 50.00 | 50.00 | 50.00 | 50.00 | 50.00 |

(continued)

| | Granulate (Reference) | | | | |
|---|---|---|---|---|---|
| | **35a** | **36a** | **37a** | **38a** | **39a** |
| Components (mg) | | | | | |
| Microcrystalline cellulose | 50.00 | - | - | - | 35.00 |
| Mannitol | - | 50.00 | - | - | - |
| Lactose monohydrate | - | - | 50.00 | - | 15.00 |
| LH-B1 | - | - | - | 50.00 | - |
| Povidone | 6.00 | - | - | - | - |
| Klucel EF | - | 6.00 | - | - | 6.00 |
| Hypromellose | - | - | 6.00 | - | - |
| LH-21 | - | - | - | 6.00 | - |
| Primojel | 10.00 | - | - | - | - |
| Crospovidone | - | 20.00 | - | - | - |
| Ac-Di-Sol | - | - | 5.00 | - | - |
| Starch 1500 | - | - | - | 20.00 | - |
| Starch | - | - | - | - | 20.00 |
| Purified water | q.s. | q.s. | q.s. | q.s. | q.s. |
| Mixing/granulating device | HS | HS | HS | HS | HS |
| Drying method | DC | DC | DC | DC | DC |
| Drying temperature (°C) | 50-65 | 60 | 55-60 | 55-65 | 60-70 |

2) *Preparation of compression mixtures*

The components shown in Table 12 below were added to the respective granulates **(35a)-(44a)** obtained above and mixed in a high-shear mixer (HSM) or container mixer (CM) to obtain compression mixture(s). The nature of the added components as well as the type of the mixer are shown in Table 12.

**Table 12.**

| | Compression mixture | | | | |
|---|---|---|---|---|---|
| | **35b** | **36b** | **37b** | **38b** | **39b** |
| Granulate | **35a** | **36a** | **37a** | **38a** | **39a** |
| Components (mg) | | | | | |
| Microcrystalline cellulose | 71.00 | - | - | - | 40.00 |
| Mannitol | - | 61.00 | - | - | - |
| Lactose monohydrate | - | - | 76.00 | - | 21.00 |
| LH-B1 | 10.00 | - | - | 71.00 | - |
| LH-11 | - | 10.00 | - | - | - |
| LH-21 | - | - | 10.00 | - | - |
| LH-22 | - | - | - | - | - |
| Primojel | - | - | - | - | 10.00 |
| Crospovidone | - | - | - | - | - |

(continued)

| Components (mg) | | | | | |
|---|---|---|---|---|---|
| Ac-Di-Sol | - | - | - | - | - |
| Starch 1500 | - | - | - | - | - |
| Magnesium stearate | 3.00 | 3.00 | 3.00 | 3.00 | 3.00 |
| Mixing device | HSM | CM | HSM | CM | HSM |

*3) Preparation of tablets*

The compression mixtures **(35b)-(39b)** were compressed into round or oval tablets with a theoretical weight of 200 mg.

**Examples 40-46: Non-aqueous moisture activated granulation**

***1)*** *Preparation of granulates*

The respective solid components shown in Table 13 below were homogenized in a high shear (HS) mixer or in a fluid bed granulator (FBG), and non-aqueous granulating liquid was sprayed over this mixture. Alternatively, a part or a whole amount of binder can optionally be dissolved into the granulation liquid which is sprayed onto the powder mixture shown in Table 1 below. The obtained granulate was optionally dried in a drying chamber (DC) or a fluid-bed dryer (FBD).

**Table 13: Composition of granulate**

| | **40a** | **41a** | **42a** | **43a** | **44a** |
|---|---|---|---|---|---|
| Components (mg) | | | | | |
| Vildagliptin | 50.00 | 50.00 | 50.00 | 50.00 | 50.00 |
| Low-substituted hydroxypropylcellulose | 13.00 | 13.00 | 13.00 | 13.00 | - |
| Hydroxypropylcellulose | - | - | - | 13.00 | 15.00 |
| Mannitol | 35.00 | 33.00 | | | 52.00 |
| Lactose, anhydrous | - | - | 35.00 | 20.00 | - |
| Colloidal silicon dioxide | | | - | 1.00 | 0.50 |
| Polyethyleneglycol 400 | 2.00 | 4.00 | - | - | - |
| Polysorbate | - | - | 2.00 | - | - |
| Propylene glycol | - | - | - | 3.00 | - |
| Glycerol | - | - | - | - | 2.50 |
| Weight of intragranular phase | 100.00 | 100.00 | 100.00 | 100.00 | 120.00 |

*Preparation of compression mixtures*

The components shown in Table 14 below were added to the respective granulates **(40a)-(44a)** obtained above and mixed in a high-shear mixer (HSM) or container mixer (CM) to obtain compression mixture(s)

**Table 14: Composition of tablets**

| | Compression mixture | | | | |
|---|---|---|---|---|---|
| | **40b** | **41b** | **42b** | **43b** | **44b** |
| Granulate | 40a | 41a | 42a | 43a | 44a |

(continued)

| Components (mg) | | | | | |
|---|---|---|---|---|---|
| Granulate | 100.00 | 100.00 | 100.00 | 100.00 | 120.00 |
| Microcrystalline cellulose | 47.00 | 46.00 | 55.00 | 46.00 | 24.00 |
| Lactose anhydrous | - | - | - | - | 40.00 |
| Sodium starch glycolate | 8.00 | 8.00 | - | - | 10.00 |
| Croscarmellose sodium | - | - | 10.00 | 8.00 | - |
| Colloidal silicon dioxide | 2.25 | 2.25 | 1.75 | 2.25 | 2.00 |
| Magnesium stearate | 2.75 | 3.75 | 3.25 | 3.75 | 4.00 |
| Total mass of tbl. | 160.00 | 160.00 | 170.00 | 160.00 | 200.00 |

3) Preparation of tablets

The compression mixtures **(40b)-(44b)** were compressed into round or oval tablets

Example 45:

Composition of solid components except a hypromellose of granulate according to Example 8a were mixed in high shear mixer or in fluid bed granulator and were granulated with a solution of hypromellose in ethanol. Obtained wet mass is optionally dryed and sieved. The obtained granulate was mixed with extragranular excipients according to composition of Table 2 and compressed into tablets.

Example 46:

Composition of solid components of granulate according to Example 43a except a hyprolose, which divided into two portions - one was admixes to the rest os sold components of the granulate and the rest was dissolved in the propyleneglycole, was mixed in high shear mixer or in fluid bed granulator and was granulated with a solution of a part of hypromellose in propylene glycol. Obtained wet mass is optionally dryed and sieved. The obtained granulate was mixed with extragranular excipients according to composition of Table 2 and compressed into tablets.

## Claims

**1.** A process of making a granulate comprising vildagliptin or an acid addition salt thereof, which process comprises:

a) providing a material comprising or consisting of vildagliptin or an acid addition salt thereof,
b) mixing the material comprising or consisting of vildagliptin or an acid addition salt thereof with one or more additional ingredients to produce a mixture comprising vildagliptin or an acid addition salt thereof,
c) subjecting the mixture comprising vildagliptin or an acid addition salt thereof to granulation,

which granulation is wet granulation with liquid(s) free of gelatine,
said wet granulation using solvent selected from alcohols with 1 to 4 carbon atoms, and mixtures thereof,
the binder for use in wet granulation being selected from hydroxymethylcellulose, hydroxyethylcellulose, hydroxypropylcellulose, and mixtures thereof.

**2.** The process according to claim 1, which process further comprises,
compressing the granulate comprising vildagliptin or an acid addition salt thereof in one or more comprimate(s), or formulating the granulate comprising vildagliptin and/or an acid addition salt thereof in one or more pellet(s), optionally coating the comprimate(s) or pellet(s), and optionally filling the comprimate(s), the pellet(s), the optionally coated comprimate(s), or the optionally coated pellet(s) into capsule(s) or sachet(s).

**3.** Granulate comprising vildagliptin or an acid addition salt thereof, said granulate being obtainable according to the process of claim 1.

**4.** Pharmaceutical composition comprising or consisting of granulate(s) according to claim 3 or comprising or consisting of comprimate(s) and/or coated comprimate(s) obtainable according to claim 2 or comprising or consisting of pellet(s) and/or coated pellet(s) obtainable according to claim 2.

**5.** Pharmaceutical composition according to claim 4, which pharmaceutical composition is a solid oral dosage form selected from the group consisting of tablets, microtablets and capsules filled with one or more of granulate(s) according to claim 3, comprimate(s) and/or coated comprimate(s) obtainable according to claim 2, and pellet(s) and/or coated pellet(s) obtainable according to claim 2.

## Patentansprüche

**1.** Verfahren zum Herstellen eines Granulats umfassend Vildagliptin oder ein Säureadditionssalz davon, wobei das Verfahren umfasst:

a) Bereitstellen eines Materials umfassend oder bestehend aus Vildagliptin oder einem Säureadditionssalz davon,
b) Mischen des Materials umfassend oder bestehend aus Vildagliptin oder einem Säureadditionssalz davon mit einem oder mehr zusätzlichen Bestandteilen, wobei ein Gemisch umfassend Vildagliptin oder ein Säureadditionssalz davon hergestellt wird,
c) Unterwerfen des Gemisches umfassend Vildagliptin oder ein Säureadditionssalz davon einer Granulierung,

wobei die Granulierung Feuchtgranulierung mit Flüssigkeit(en), frei von Gelatine, ist, wobei die Feuchtgranulierung Lösungsmittel ausgewählt aus Alkoholen mit 1 bis 4 Kohlenstoffatomen und Gemischen davon verwendet,
wobei das Bindemittel zur Verwendung beim Feuchtgranulieren aus Hydroxymethylcellulose, Hydroxyethylcellulose, Hydroxypropylcellulose und Gemischen davon ausgewählt ist.

**2.** Verfahren gemäß Anspruch 1, wobei das Verfahren ferner umfasst
Komprimieren des Granulats umfassend Vildagliptin oder ein Säureadditionssalz davon in ein oder mehr Komprimat(e) oder
Formulieren des Granulats umfassend Vildagliptin und/oder ein Säureadditionssalz davon in ein oder mehr Pellet(s),
gegebenenfalls Überziehen des Komprimats oder Pellets beziehungsweise der Komprimate oder Pellets, und gegebenenfalls Füllen des Komprimats, des Pellets, des gegebenenfalls überzogenen Komprimats oder des gegebenenfalls überzogenen Pellets beziehungsweise der Komprimate, der Pellets, der gegebenenfalls überzogenen Komprimate oder der gegebenenfalls überzogenen Pellets in Kapsel(n) oder Portionspackung(en).

**3.** Granulat umfassend Vildagliptin oder ein Säureadditionssalz davon, wobei das Granulat gemäß dem Verfahren von Anspruch 1 erhältlich ist.

**4.** Pharmazeutische Zusammensetzung umfassend oder bestehend aus Granulat(en) gemäß Anspruch 3 oder umfassend oder bestehend aus gemäß Anspruch 2 erhältlichem Komprimat(en) und/oder überzogenem Komprimat(en) oder umfassend oder bestehend aus gemäß Anspruch 2 erhältlichem Pellet(s) und/oder überzogenem Pellet(s).

**5.** Pharmazeutische Zusammensetzung gemäß Anspruch 4, wobei die pharmazeutische Zusammensetzung eine feste orale Dosierungsform ist, ausgewählt aus der Gruppe bestehend aus Tabletten, Mikrotabletten und Kapseln, gefüllt mit einem oder mehr von Granulat(en) gemäß Anspruch 3, gemäß Anspruch 2 erhältlichem Komprimat(en) und/oder überzogenem Komprimat(en) und gemäß Anspruch 2 erhältlichem Pellet(s) und/oder überzogenem Pellet(s).

## Revendications

**1.** Procédé de fabrication d'un granulé comprenant de la vildagliptine ou un sel d'addition d'acide de celle-ci, lequel procédé comprend le fait :

a) de fournir un matériau comprenant ou consistant en la vildagliptine ou un sel d'addition d'acide de celle-ci,
b) de mélanger le matériau comprenant ou consistant en la vildagliptine ou un sel d'addition d'acide de celle-ci avec un ou plusieurs ingrédient(s) supplémentaire(s) pour produire un mélange comprenant de la vildagliptine ou un sel d'addition d'acide de celle-ci,

c) de soumettre le mélange comprenant de la vildagliptine ou un sel d'addition d'acide de celle-ci à une granulation,

laquelle granulation est une granulation par voie humide avec un/des liquide(s) exempt(s) de gélatine, ladite granulation par voie humide utilisant un solvant choisi parmi des alcools ayant 1 à 4 atome(s) de carbone et des mélanges de ceux-ci,
le liant pour une utilisation dans la granulation par voie humide étant choisi parmi l'hydroxyméthylcellulose, l'hydroxyéthylcellulose, l'hydroxypropylcellulose et des mélanges de celles-ci.

**2.** Procédé selon la revendication 1, lequel procédé comprend en outre le fait :
de comprimer le granulé comprenant de la vildagliptine ou un sel d'addition d'acide de celle-ci en un ou plusieurs produit(s) comprimé(s), ou de formuler le granulé comprenant de la vildagliptine et/ou un sel d'addition d'acide de celle-ci en une ou plusieurs pastille(s), d'enrober facultativement le/les produit(s) comprimé(s) ou la/les pastille(s), et d'introduire facultativement le/les produit(s) comprimé(s), la/les pastille(s), le/les produit(s) comprimé(s) facultativement enrobé(s), ou la/les pastille(s) facultativement enrobée(s) dans une/des capsule(s) ou un/des sachet(s).

**3.** Granulé comprenant de la vildagliptine ou un sel d'addition d'acide de celle-ci, ledit granulé pouvant être obtenu selon le procédé de la revendication 1.

**4.** Composition pharmaceutique comprenant ou consistant en un/des granulé(s) selon la revendication 3 ou comprenant ou consistant en un/des produit(s) comprimé(s) et/ou un/des produit(s) comprimé(s) enrobé(s) pouvant être obtenu(s) selon la revendication 2 ou comprenant ou consistant en une/des pastille(s) et/ou une/des pastille(s) enrobée(s) pouvant être obtenue(s) selon la revendication 2.

**5.** Composition pharmaceutique selon la revendication 4, laquelle composition pharmaceutique est une forme galénique orale solide choisie dans le groupe constitué de comprimés, de microcomprimés et de capsules remplis d'un(e) ou de plusieurs parmi un/des granulé(s) selon la revendication 3, un/des produit(s) comprimé(s) et/ou un/des produit(s) comprimé(s) enrobé(s) pouvant être obtenu(s) selon la revendication 2, et une/des pastille(s) et/ou une/des pastille(s) enrobée(s) pouvant être obtenue(s) selon la revendication 2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1137635 A
- WO 2004092127 A
- WO 2007078726 A2
- WO 2009121945 A2
- WO 2007019255 A2
- WO 2007041053 A2
- WO 0034241 A1
- CN 101234105 A
- CN 101181264 A
- EP 1620396 A
- EP 1912938 A